(19) Europäisches Patentamt
European Patent Office
Office européen des brevets

(11) **EP 3 083 956 B1**

(12) **FASCICULE DE BREVET EUROPEEN**

(45) Date de publication et mention de la délivrance du brevet:
**26.06.2019 Bulletin 2019/26**

(21) Numéro de dépôt: **14827477.2**

(22) Date de dépôt: **08.12.2014**

(51) Int Cl.:
***C12N 15/01*** *(2006.01)*  ***C12R 1/865*** *(2006.01)*
***A21D 8/04*** *(2006.01)*  ***C12N 1/18*** *(2006.01)*

(86) Numéro de dépôt international:
**PCT/FR2014/053220**

(87) Numéro de publication internationale:
**WO 2015/092208 (25.06.2015 Gazette 2015/25)**

(54) **NOUVELLES SOUCHES DE LEVURE DE PANIFICATION PERFORMANTES SUR PATES NON SUCREES OU LEGEREMENT SUCREES**

HEFESTÄMME FÜR EFFICIENTES BACKEN VON UNZUCKERTEN ODER WENIG GEZUCKERTEN TEIG

YEAST STRAINS FOR EFFICIENT BREAD MAKING FROM NON OR LOW SUGARED DOUGH

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.12.2013 FR 1362644**

(43) Date de publication de la demande:
**26.10.2016 Bulletin 2016/43**

(73) Titulaire: **Lesaffre et Compagnie**
**75001 Paris (FR)**

(72) Inventeurs:
• **BARTOLUCCI, Jean-Charles**
**F-59260 Hellemmes (FR)**
• **FONCHY-PENOT, Evelyne**
**F-62840 Fleurbaix (FR)**
• **LAGOUTTE YALCIN, Ilknur**
**F-59700 Marcq-en-Baroeul (FR)**
• **PARASIE, Georges**
**F-59320 Sequedin (FR)**
• **PETROFF, Dominique**
**F-92190 Meudon (FR)**
• **QUIPOURT-ISNARD, Anne-Dominique**
**F-59700 Marcq-en-Baroeul (FR)**
• **TRIONE, Valérie**
**F-59560 Comines (FR)**

(74) Mandataire: **Cabinet Plasseraud**
**66, rue de la Chaussée d'Antin**
**75440 Paris Cedex 09 (FR)**

(56) Documents cités:
EP-A1- 0 511 108    WO-A1-97/28693
WO-A1-2012/110711   WO-A1-2014/060678
FR-A1- 2 920 157

## Description

### Domaine de l'invention

La présente invention se rapporte à des nouvelles souches de levure de panification *Saccharomyces cerevisiae* performantes sur pâtes non sucrées et/ou légèrement sucrées. Elle se rapporte aussi aux levures obtenues par multiplication des dites souches ainsi qu'à leur utilisation pour la production de produits cuits de boulangerie.

### Arrière-plan technologique

La consommation des produits de panification non sucrés et/ou légèrement sucrés est en constante progression. La fabrication de ces produits met en oeuvre des levures de panification, dites de boulangerie, sous forme sèche, pressée ou liquide.

Il existe des souches de levure de panification adaptées aux différents types de pâtes : non sucrées, légèrement ou fortement sucrées. Ainsi, la demanderesse propose une gamme complète de levures destinées à tout type de panification et à tout produit cuit de boulangerie.

Cependant, les levuriers sont constamment à la recherche de nouvelles souches de levures permettant de produire des levures encore plus performantes notamment en termes de forces fermentatives et de conservation.

La demande internationale publiée sous la référence WO2012/110711 décrit par exemple des souches de *Saccharomyces cerevisiae* aptes à la production de levures de panification osmo-tolérantes et présentant une résistance intrinsèque aux acides organiques faibles.

Les levuriers sont aussi à la recherche permanente de procédé amélioré de production de levure. Comme par exemple l'amélioration de l'économie de production qui peut être atteinte par une productivité plus élevée, par la réduction du coût de production, par la facilité de mise en oeuvre tout au long des processus de production de levure quelle qu'en soit la forme : sèche, pressée ou liquide.

La robustesse et la régularité de la souche ainsi que sa résistance au séchage sont aussi des propriétés que les levuriers recherchent.

Une des souches références de la demanderesse dans ce domaine est celle déposée le 12 février 2003 à la CNCM (Collection Nationale de Cultures de Microorganismes, 26 rue du Docteur Roux, 75724 Paris cedex 15) sous le numéro I-2970. Il s'agit d'une souche importante ayant des applications multiples en levure sèche, en levure pressée et en levure liquide. Toute amélioration de cette souche impacte donc plusieurs produits et qualités.

Pour une souche donnée et au vu des conditions d'environnement optimisées notamment en termes de pH, de température, du milieu de fermentation, des sources en azote (N) et en phosphore (P), le paramètre le plus important est la cinétique de croissance caractérisée par l'évolution des taux de multiplication horaire et plus globalement le taux de multiplication moyen TMm.

Le taux de multiplication moyen se définit par $\sqrt[d]{(Xfin/X0)}$ , avec

- d= durée de la fermentation
- Xfin la quantité de levure en fin de fermentation
- X0 la quantité de levure au début de la fermentation

Il est connu qu'augmenter le taux de multiplication moyen a comme effet l'augmentation de la capacité fermentaire d'une levure au détriment d'une perte de productivité.

En effet, en conditions industrielles de fermentation pour produire la levure de boulangerie, le taux de croissance est contrôlé par le débit d'alimentation en sucre selon une technologie de fermentation semi-continue (Fed-Batch) et en conditions d'aérobiose ce qui permet d'optimiser et le rendement de production de la biomasse et la productivité du fermenteur.

Les limites du procédé industriel concernant le taux de multiplication moyen TMm interviennent à plusieurs niveaux :
Sur le premier tiers de la fermentation, le taux de multiplication horaire est piloté de façon à rester inférieur au taux de multiplication critique pour éviter le métabolisme Respiro fermentatif associé à la production d'éthanol qui pénalise le rendement de production de la biomasse levure à partir du sucre.

Sur la suite de la fermentation, au fur et à mesure que la biomasse levure augmente dans le fermenteur, le taux de multiplication horaire est réduit progressivement pour ne pas être en limite des capacités de transfert d'oxygène et de refroidissement du fermenteur industriel.

Globalement plus les taux de multiplication horaire sont élevés et plus le métabolisme permet d'obtenir des levures riches en protéines dont les enzymes conférant le pouvoir fermentaire de la levure.

Si l'on recherche une levure à très haute activité fermentaire, il est nécessaire d'appliquer des taux de multiplication horaire plus élevés, donc des vitesses de consommation en $O_2$ plus élevées et des vitesses de production de calories plus élevées (procédé rapide). En conséquence les capacités de transfert en Oxygène et refroidissement du fermenteur étant ce qu'elles sont, il est nécessaire de réduire la quantité de levure dans le fermenteur et donc de perdre en productivité.

Un des problèmes que cherche à résoudre l'invention est donc de disposer au moins d'une nouvelle souche de levure ayant une activité fermentaire améliorée par rapport à la souche de référence I-2970 tout en étant produite avec un faible taux de multiplication moyen (cinétique lente) et une productivité compatible avec un usage industriel et commercial.

Après de multiples essais de sélection de souches, la demanderesse a identifié et sélectionné une nouvelle souche ayant une excellente activité fermentaire tout en étant produite en fermentation avec une cinétique de croissance lente, ce qui a permis de résoudre le problème évoqué ci-dessus.

## Résumé de l'invention

L'invention a donc pour objet des nouvelles souches de levures qui, produites en fermentation avec une cinétique de croissance plus lente que celle de la souche de référence I-2970, permettent l'obtention de levures de boulangerie présentant une activité fermentaire en pâte non sucrée ou légèrement sucrée au moins égale à celle de la souche de référence.

En particulier, l'invention a comme objet la souche déposée le 25 avril 2013 à la Collection Nationale de Cultures de Microorganismes, 26 rue du Docteur Roux, 75724 Paris cedex 15 sous le numéro I-4743.

L'invention a aussi pour objet les levures de boulangerie, sous forme sèches, pressées ou liquides, obtenues par multiplication de la souche I-4743.

Par l'expression « souche dérivée », on désigne une souche dérivée par toute transformation quelle qu'elle soit, comme par exemple par un ou plusieurs croisements et/ou par mutation et/ou par transformation génétique.

Une souche dérivée par croisement peut être obtenue par croisement d'une souche selon l'invention avec la même souche, ou avec une autre souche selon l'invention, ou avec une autre souche quelconque.

Une souche dérivée par mutation peut être une souche qui a subi au moins une mutation spontanée dans son génome ou au moins une mutation induite, par exemple par mutagenèse. La ou les mutations de la souche dérivée sont silencieuses ou non.

Par l'expression « mutagenèse », on désigne à la fois la mutagenèse classique obtenue par rayonnements, par exemple par l'utilisation d'UV, ou par des agents chimiques mutagènes et la mutagenèse insertionnelle par transposition ou par intégration d'un fragment d'ADN exogène.

La mutagenèse par rayonnements comprend l'utilisation de rayonnements UV, X, ou gamma.

Les agents chimiques mutagènes sont par exemple l'EMS (éthyl-méthyl sulfonate), l'EES (éthyl-éthyl sulfonate), la nitrosoguanidine, l'acide nitreux, l'aflatoxine B1, l'hydroxylamine, la 5-bromo uracile, la 2-amino purine, la proflavine, l'acridine orange.

Une souche dérivée par transformation génétique est une souche dans laquelle a été introduit un ADN exogène. Cet ADN exogène est de préférence apporté par un plasmide ou intégré directement dans le génome.

L'invention a aussi comme objet les pâtes boulangères obtenues par un procédé comprenant une étape de fermentation d'une levure de boulangerie selon l'invention, ainsi que les produits cuits de boulangerie obtenus.

## Description détaillée de l'invention

L'objet de la présente invention est de fournir au moins une souche de levure de boulangerie, donnant après multiplication industrielle, une levure de panification possédant une activité fermentaire dans des pâtes sans sucre ou légèrement sucrées supérieure ou égale à celle obtenue avec la souche de référence I-2970 tout en suivant une cinétique de croissance plus lente que celle de la souche de référence.

Les levures obtenues par ce procédé de production dit lent peuvent être présentées sous différentes formes : sèches, pressées ou liquides. Elles sont résistantes aux conditions de production, notamment au séchage, et stables au stockage.

Par pâte légèrement sucrée (PS) on entend une pâte boulangère comprenant moins de 10% de sucre ajouté.

Par procédé de production dit rapide, appliqué dans l'étape finale de fermentation industrielle, on entend un taux de multiplication moyen supérieur ou égal à 1,17.

Par procédé de production lent appliqué dans l'étape finale de fermentation industrielle, on entend un taux de multiplication moyen inférieur ou égal à 1,15.

La résistance au séchage se traduit par le maintien après séchage d'une activité fermentaire au moins égale à 70% de l'activité fermentaire avant séchage.

Les avantages des souches de l'invention se manifestent aussi lorsque les levures obtenues par culture de la dite souche sont utilisées comme agent de fermentation dans des pâtes sans sucre ou légèrement sucrées.

La souche I-2970 est considérée par la demanderesse comme souche référence dans le domaine intéressant la présente invention. Dans sa recherche de solution au problème évoqué précédemment, la demanderesse s'est naturellement intéressée et à cette souche, comme point de départ, ainsi qu'aux souches dérivées par mutation de celle-ci.

La demanderesse a, tout d'abord, fait subir à la souche de référence une mutation notamment par UV suivie d'une sélection rigoureuse et motivée parmi les milliers de mutants obtenus.

Et c'est ainsi que la souche I-4743 a été sélectionnée par la demanderesse comme étant la meilleure solution aux problèmes évoqués précédemment.

Le procédé de sélection mis au point par la demanderesse est basé, entre autres, sur les critères suivants pris séparément ou en combinaison :

- aptitude de la souche à assimiler l'azote présent dans le milieu de culture
- quantité de biomasse produite,
- pouvoir ferment de la levure obtenue,
- perte de rendement de croissance sur sucre.

Le procédé de sélection appliqué à la souche de référence I-2970 comprend au moins les étapes de sélection suivantes :

Etape I

I.1) Mutagenèse de la souche de référence I-2970 permettant l'obtention de 10 000 clones mutants,

I.2) criblage des 10 000 clones : deux criblages réalisés à l'échelle de la microplaque (évaluation par dosage de l'éthanol en milieu liquide) et deux criblages réalisés à l'échelle de la fiole. Les critères de sélection retenus dans cette étape sont :

- la quantité d'éthanol produite sur milieu synthétique liquide mimant une pâte normale doit être supérieure ou égale à la quantité d'éthanol produite par la souche de référence,
- la quantité de biomasse produite dans le milieu de culture doit être supérieure ou égale à la quantité de biomasse produite par la souche référence I-2970, et
- la force fermentative en pâte sans sucre doit être supérieure d'au moins 10% à la force fermentative de la souche de référence I-2970.

Le milieu de culture développé spécifiquement pour le criblage des souches est un milieu riche en molécules azotées et ceci dans le but d'éliminer dès ce premier criblage tous les mutants n'ayant pas une bonne aptitude à assimiler l'azote.

L'étape I.2 permet la sélection de 40 à 50 clones.

Etape II

II.1) Les clones retenus dans l'étape I.2 sont étudiés en fermenteur de 1 litre. Les critères de sélection sont :

- la perte de rendement de croissance sur sucre ne doit pas être supérieure à 10% par rapport au rendement de croissance de la souche de référence I-2970,
- la force fermentative en PN qui doit être supérieure d'au moins 10% à celle de la souche référence.

Ainsi une dizaine de clones répondant aux critères ci-dessus ont été sélectionnés.

II.2) Etude en fermenteur de 7 litres des clones retenus dans l'étape II.1. Les critères de sélection sont :

- le rendement de croissance sur sucre doit être au moins équivalent au rendement obtenu avec la souche de référence I-2970,
- la force fermentative doit être supérieure d'au moins 10% à celle de la souche de référence, et

- la teneur en azote de la levure doit être supérieure d'au moins 5% à celle de la souche de référence.

Ainsi, un à deux clones répondant aux critères ci-dessus ont été sélectionnés.

Etape III

III.1) Etude des clones sélectionnés en II.2 en fermenteur de 20 litres. Cela inclut une propagation de la levure mère en deux générations comme cela est pratiqué en usine. La levure mère sert à ensemencer la fermentation finale dite commerciale, la souche de référence I-2970 étant produite dans les mêmes conditions. En fin de fermentation commerciale la levure est séparée, déshydratée. Les critères d'évaluation à cette étape sont :

- L'absence d'anormalité de la fermentation au vu des paramètres classiquement étudiés (cinétique, besoins nutritifs, rendement de croissance sur sucre) ;
- La force fermentative qui doit être supérieure de 10% à celle de la souche de référence I-2970 ;
- La teneur en azote qui doit être supérieure de 5% à celle de la souche de référence I-2970.

La levure déshydratée et séchée sous la forme de levure sèche instantanée désignée généralement par SPI, est ensuite évaluée.

Le critère d'évaluation est la perte de force fermentative au séchage qui doit être inférieure ou égale à 30%.

La levure sèche est aussi évaluée dans différentes recettes de panification par la mesure du proof time. Le critère de sélection est un proof time qui doit être supérieur de 5% à celui de la souche référence.

Le procédé de sélection ci-dessus a permis la sélection de la souche de levure de boulangerie déposée le 25 avril 2013 à la Collection Nationale de Cultures de Microorganismes, 26 rue du Docteur Roux, 75724 Paris cedex 15 sous le n° I-4743 qui constitue le premier objet de la présente invention.

Le procédé de sélection mentionné ci-dessus appliqué aux souches dérivées de la souche I-4743 permet de sélectionner aussi d'autres souches permettant de disposer de nouvelles souches ayant une activité fermentaire améliorée ou du moins identique à celle de la souche de référence I-2970 lorsque les souches sont produites en fermentation en procédé lent.

Un autre objet de la description est donc une souche dérivée de la souche I-2970 ou de la souche I-4743 caractérisée en ce qu'elle répond aux critères de sélections des étapes I, II et III décrites précédemment.

Les souches de levure de l'invention sont utilisées pour la fabrication de levures de panification comme décrit dans le manuel « Yeast Technology », 2ème édition, 1991, G. Reed et T.W. Nagodawithana, publié par Van Nostrand Reinhold, ISBN 0-442-31892-8.

La fabrication de levure de boulangerie comprend au moins les deux premières étapes de l'ensemble d'étapes suivantes :

- multiplication d'une souche pure de levure de boulangerie en plusieurs stades, d'abord en semi-anaérobiose, puis en aérobie,
- séparation par centrifugation de la levure de boulangerie ainsi produite de son milieu de culture, avec l'obtention d'une « crème de levure » liquide contenant environ entre 14 et 25% de matières sèches, filtration de la crème de levure liquide ainsi obtenue, en général après salage, sur un filtre rotatif sous vide et obtention d'une levure fraîche déshydratée contenant environ 26 à 35% de matières sèches,
- malaxage de ladite levure fraîche déshydratée en vue de l'obtention d'une masse bien homogène,
- extrusion de la levure ainsi obtenue, soit sous forme de pains de levure fraîche pressée, soit sous forme de levure fraîche émiettée, commercialisées à environ 30% de matières sèches, soit sous forme de fins filaments si la levure est destinée à être séchée.
- si la levure est séchée, le séchage est de préférence un séchage rapide ménageant en présence d'émulsifiant, notamment le monostéarate de sorbitan (MSS) à 1,5%. Le séchage se fait dans un courant d'air chaud, par exemple par fluidisation, des particules de levures obtenues par extrusion,
- emballage sous vide de la levure sèche.

La levure de l'invention peut être présentée sous la forme de crèmes de levure, de levures pressées et de levures sèches

De manière générale, les levures obtenues par culture de la souche de l'invention présentent:

a. un temps d'apprêt en panification inférieur à celui obtenu avec les levures de panification issues de la souche I-2970, et/ou

b. une résistance au séchage supérieure ou égale à celle de la souche I-2970.

De plus, elles présentent une force fermentative en pâte légèrement sucrée supérieure de 2 à 6% et de préférence de 3 à 5 % à celle obtenue avec la souche de référence I-2970.

Un autre objet de l'invention est un procédé de préparation de pâte boulangère non sucrée ou légèrement sucrée mettant en oeuvre une étape de fermentation d'une levure telle que celle de l'invention ainsi que les pâtes et les produits de boulangerie cuits obtenus.

Les exemples suivants illustrent l'invention sans en limiter la portée.

## EXEMPLES :

Les souches étudiées ont été évaluées en fermentation par leur croissance caractérisée par le taux de multiplication moyen, et les levures produites sont évaluées par leur teneur en azote, par leur force fermentative ainsi que par le rapport force fermentative /teneur en azote.

La force fermentative correspond au volume de $CO_2$ (en ml) produit par la levure en fermentation dans des pâtons de farine. La force fermentative est mesurée par des techniques classiques connues de l'homme du métier, notamment au moyen d'un fermentomètre comme décrit par Burrows et Harrison dans « Journal of the Institute of Brewing », Vol 65, 1959. En particulier, la force fermentative est mesurée selon des tests décrits dans EP0511108 et US5741695 au nom de la demanderesse.

La force fermentative est mesurée sur des pâtons constitués de 20g de farine et d'une suspension de levures, dans un fermentomètre de type Burrows et Harrison, sur une durée de 2 heures.

Pour la mesure de la force fermentative après culture en milieu riche en molécules azotées, la suspension de levures est constituée de 100 mg de matières sèches de levure dans 15 ml d'eau contenant 27g/l de NaCl et 4g/l de SO4(NH4)2.

Pour la mesure de la force fermentative après culture semi-continue, la suspension de levures est constituée de 150 mg de matières sèches de levure dans 15 ml d'eau contenant 27g/l de NaCl et 4g/l de SO4(NH4)2.

Pour constituer les pâtons, le mélange de farine et de suspension de levures est malaxé pendant 40 secondes dans un pétrin, de manière à obtenir une pâte qui est ensuite placée dans un bain-marie à 30°C. 13 minutes après le malaxage, le récipient contenant la pâte est fermé hermétiquement. La quantité totale de gaz produite est mesurée en ml à 2 heures à 30°C.

La force fermentative est mesurée dans différentes conditions de pâte :

- pâte sans sucre dont la composition est celle mentionnée ci-dessus (PN),

- pâte sucrée contenant 2g de saccharose pour 20g de farine (PS), et

La différence de force fermentative des souches testées par rapport à celle de la souche de référence en pourcentage est calculée selon la formule suivante :

$$\frac{[(\text{force fermentative de la souche testée}) - (\text{force fermentative de la souche de référence})}{(\text{force fermentative de la souche de référence})} \times 100$$

La teneur en azote est déterminée par la méthode de Kjeldahl.

### Exemple 1 :

Mutagenèse et premiers criblages

Le milieu de culture développé spécifiquement pour le criblage est un milieu riche en molécules azotées et ceci dans le but de vérifier la capacité des souches sélectionnées à assimiler une quantité importante d'azote.

Les critères appliqués pour la sélection sont :

- la production d'éthanol sur milieu synthétique liquide mimant une pâte normale. Les quantités d'éthanol produites par chaque mutant sont comparées avec celle produite par la souche de référence,
- la production de $CO_2$ dans une pâte normale sans sucre comprenant 100 mg de levure exprimée en matière sèche, pour 20g de farine en comparant les quantités de $CO_2$ produites par chaque mutant avec celle produite par la souche de référence,

- la quantité de biomasse produite sur le milieu développé pour le criblage en comparant à la souche de référence.

Cette étape a permis la sélection de 40 à 50 souches présentant une production de biomasse au moins identique à celle de la souche de référence cultivée dans les mêmes conditions et une force fermentative en pâte normale supérieure d'au moins de 10% à celle de la souche de référence.

Parmi les souches sélectionnées, la souche I-4743 se distingue par : une production de biomasse supérieure de 40% à celle obtenue avec la souche de référence I-2970 cultivée dans les mêmes conditions ainsi que par une force fermentative en pâte normale (PN) augmentée de 40% par rapport à celle de la souche de référence.

**Exemple 2**

Sélection en fermenteur de 1 litre :

Les souches sélectionnées à l'exemple 1 ont été ensuite étudiées en fermenteur 1 litre en mode Fed-Batch sur milieu synthétique pour valider leur aptitude à suivre une cinétique de fermentation représentative des levures de boulangerie.

Le schéma réalisé comprend un débit continu d'alimentation en sucre durant la fermentation et les nutriments sont apportés en pied de cuve.

Après la production des différentes souches, un bilan biomasse a été réalisé de manière à ne conserver que les souches ne présentant pas de carences nutritionnelles. La teneur en azote des levures en fin de fermentation a aussi été mesurée. Les résultats obtenus ont été ensuite croisés avec les forces fermentatives en pâte normale sans sucre.

Cette étape a permis la sélection de plusieurs souches, parmi lesquelles la souche I-4743 se distingue par :

- Une perte en rendement de croissance sur sucre inférieure à 10% par rapport au rendement de croissance de la souche de référence.

- Une force fermentative en PN supérieure à 15% à celle de la souche de référence.

Sélection en fermenteur de 7 litres :

Les meilleures souches sélectionnées suite aux essais en fermenteur de 1 litre ont été produites sur milieu mélasse en fermenteur de 7 litres suivant un schéma Fed-Batch dont le taux de multiplication moyen est de 1,141.

Cette étape a permis la sélection de plusieurs souches présentant :

- un rendement de croissance au moins égal à celui obtenu avec la souche de référence,

- une teneur en azote supérieure à 8,5% par rapport à la matière sèche levure (MSL),

- une force fermentative sur pâte normale sans sucre ramenée à la teneur en azote de la levure (PN/N) supérieure à 5% à celle obtenue avec la souche de référence.

De ces essais, il ressort que la levure produite avec la souche I-4743 a un rendement de croissance sur sucre quasi-équivalent à la souche de référence et se distingue par ses forces en PN et en pâte sucrée 2g nettement supérieures à la souche I-2970 (+10% en PN et +3% en PS2g). Par ailleurs, cette levure a une teneur en azote plus élevée (~9%/MSL) que la souche de référence. Cette remarque renforce l'idée que cette souche a un métabolisme de synthèse de protéines amélioré. La souche I-4743 présente aussi un net avantage par rapport à la souche de référence en terme de pouvoir ferment sur pâte normale ramené à la teneur en azote PN/N (PN/N de 21,7 vs 20,6 soit + de 5%).

**Exemple 3**

La souche I-4743 a ensuite été testée en fermenteur de 20 litres selon le mode suivant :

- propagation de la levure mère en deux générations. La levure mère ensemence toute une série de fermentations de levure commerciale qui sont réalisées pour obtenir des levures avec différents taux de protéines en les cultivant soit sur un schéma rapide soit sur un schéma lent.

- suivi des paramètres de fermentation classiquement étudiés (croissance, besoins nutritifs, composition finale de la levure, rendement de croissance sur sucre),

- évaluation de la qualité des levures commerciales en mesurant la force fermentative PN au fermentomètre séparation, salage de la crème, déshydratation sur filtre rotatif,

- la levure déshydratée est homogénéisée avec une émulsion composée de MSS 12,5% et d'huile 6%, de façon à obtenir un taux de 1,3% de MSS sur la levure sèche finale. Après extrusion sur une grille de perforation de 0,6mm, la levure subie un séchage discontinu à air fluidisé, sur séchoir glatt avec température de phase 1 de 55°C avec de l'air séché à 2g d'eau/kg AS, débit d'air de 30 m3/h et température de l'air en phase 2 de 48°C, débit d'air de 30m3/h. La levure sèche est ensuite emballée sous vide,

- évaluation de la qualité de la levure sèche par mesure de la MS et par la force fermentative dans le test fermentomètre PN et PS 2g. Evaluation de la conservation de la levure sèche dans un test 14j 43°C (conservation pendant 14 jours à 43 °C) en suivant la force fermentative PN au fermentomètre.

Le témoin est une pâte obtenue dans les mêmes conditions et avec une même composition de pâte, si ce n'est qu'elle est ensemencée avec la levure fabriquée dans les mêmes conditions que les souches testées, mais à partir de la souche de référence I-2970.

Les résultats obtenus montrent que :

- si l'on applique un schéma avec un taux moyen de multiplication lent (1,150), la souche I-4743 a une teneur en azote plus élevée que celle de la souche de référence. Ainsi I-4743 peut atteindre une teneur en azote de 9% alors qu'en comparaison la souche de référence a pour ce type de cinétique lente des difficultés à atteindre une teneur en azote de 8,3%, ce qui se traduit par une force fermentative améliorée.

- le ratio force fermentative en pâte normale / teneur en azote (PN/N) permet de comparer la capacité fermentaire entre les deux souches. Le ratio de la souche I-4743 est supérieur à celui de la souche I-2970 de plus de 10% (PN/N de 22 vs 19,5).

Ceci montre que sur l'aspect quantitatif, la synthèse de protéines de la souche I-4743 est supérieure à celle de la souche I-2970.

Les opérations de post-traitement pour obtenir une levure sèche de bonne qualité se sont déroulées normalement. :

- La taille des cellules n'est pas plus petite que la souche de référence, ce qui ne pénalise pas le rendement de la séparation, ni l'étape de déshydratation sur le filtre rotatif sous vide à précouche avec une levure déshydratée normale avec 31 -32% MS.

- La levure après ajout de l'émulsion huile/MSS n'est pas collante ni grasse, et donc ne pénalise pas l'étape d'extrusion des vermicelles.

- Pas d'allongement de la durée du séchage pour obtenir une teneur finale en %MS correcte de 95,5%.

- Pas de couleur ni d'odeur anormale observée sur la levure sèche à l'état frais et après conservation.

- La levure sèche fabriquée avec la souche I-4743 avec une cinétique de fermentation lente, présente une force fermentative sur PN améliorée par rapport à celle obtenue avec la souche I-2970 (+10% en moyenne avec un maximum de +22%).

- Bonne aptitude au séchage de la souche I-4743 car les pertes de force au séchage sont de même niveau que la souche I-2970, soit de -19% à -15% selon les tests sans sucre ou avec un peu de sucre.

Force fermentative mesurée sur la levure sèche après conservation :

Les pertes de force liées à la conservation (14 jours à 43°C) sont comparables entre les deux souches : 15% de perte de force après 14 jours de conservation à une température de 43 °C.

Evaluation de la souche en panification :

Les levures sèches produites avec la souche I-4743 et I-2970 ont été évaluées en panification, selon les conditions de mise en oeuvre suivantes.

La recette employée est exprimée en pourcentages boulangers, à savoir en masse d'ingrédients (en g) pour 100g de farine :

| Farine | 100% |
|---|---|
| Eau | 65% |
| Sel | 2% |
| Levure Sèche | 1,2% |
| Améliorant de panification | 1% |

Les essais sont réalisés dans un fournil à 22°C, avec une farine tempérée à 22°C.

Le protocole d'essai appliqué est le suivant :

- Les ingrédients secs sont placés dans une cuve Mac Duffy® d'un pétrin Hobart A200®, dont la double enveloppe est préalablement thermostatée à 21,5°C, et mélangés en première vitesse 1 minute.

- L'eau de coulage à 20°C est incorporée au pétrin.

- On procède alors à un malaxage de 7 minutes en vitesse 1, puis à un pétrissage de 1 minute 30 secondes en vitesse 2.

- La pâte est alors immédiatement décuvée et l'on mesure sa température, qui doit être de 25°C +/-0.5°C.

- On procède directement à la division en pâtons de 320 g, puis à un boulage peu serré et on laisse reposer les boules sous une couvrante.

- 35 minutes après la fin du pétrissage, on façonne mécaniquement les pâtons, que l'on place dans des moules (dimensions : base du moule de 185x75 mm ; haut du moule de 200x90 mm ; hauteur du moule de 75 mm), eux-mêmes disposés dans un incubateur Stéricult® dont les consignes sont réglées à 35°C et 90% HRE.

- On mesure alors le « Proof Time » qui est le temps entre la mise en place des moules dans l'incubateur et le moment où la pâte atteint une hauteur de 85 mm dans le moule.

- Après une heure de pousse dans l'incubateur, une partie des moules est placée dans un four à chariot rotatif Angoulvant ventilé pendant 22 minutes à 205°C.

L'un des indicateurs les plus intéressants en termes de performance est le temps de pousse, ou Proof Time, mesuré en minutes. Dans le tableau qui suit sont indiqués les « Proof Time » obtenus avec les levures sèches produites avec les deux souches, selon que le schéma de multiplication est lent ou rapide. Nous indiquons les fourchettes de valeurs obtenues, selon différents essais.

| | | Souche | |
|---|---|---|---|
| | | I-4743 | I-2970 |
| Schéma | Lent | 75 - 81 | 84 - 86 |
| | Rapide | 72 - 81 | 78 - 83 |

Fourchettes de Proof Time obtenus lors de divers essais, en panification.

On observe qu'en schéma lent, les performances obtenues avec la souche I-4743 sont du niveau de celles obtenues avec la souche de référence I-2970, en schéma rapide.

**Revendications**

1. Souche de levure *Saccharomyces cerevisiae* déposée le 25 avril 2013 auprès de la CNCM sous le numéro I-4743.

2. Levure de boulangerie obtenue par culture de la souche selon la revendication 1, **caractérisée en ce qu'**elle est sous une forme liquide, semi liquide, pressée ou sèche.

3. Procédé de préparation d'une pâte boulangère comprenant une étape de fermentation par une levure selon la revendication 2.

4. Pâte boulangère obtenue selon le procédé de la revendication 3.

5. Pâte boulangère selon la revendication 4, **caractérisée en ce qu'**elle comprend moins de 10% de sucre ajouté.

6. Procédé de préparation d'un produit cuit de panification comprenant une étape de cuisson d'une pâte boulangère obtenue selon le procédé de la revendication 3.

7. Produit de panification susceptible d'être obtenu par le procédé selon la revendication 6.


**Patentansprüche**

1. Hefestamm *Saccharomyces cerevisiae,* eingereicht am 25. April 2013 bei der CNCM unter der Nummer I-4743.

2. Backhefe, erhalten durch Kultivieren des Stamms gemäß Anspruch 1, **dadurch gekennzeichnet, dass** sie in flüssiger, halbflüssiger, gepresster oder trockener Form vorliegt.

3. Verfahren zur Herstellung eines Backteigs, das einen Schritt umfasst, bei dem eine Hefe gemäß Anspruch 2 fermentiert wird.

4. Backteig, erhalten gemäß dem Verfahren von Anspruch 3.

5. Backteig gemäß Anspruch 4, **dadurch gekennzeichnet, dass** er mindestens 10% an zugesetztem Zucker enthält.

6. Verfahren zur Herstellung eines gebackenen Brotprodukts, umfassend einen Schritt, bei dem ein Backteig, erhalten gemäß dem Verfahren von Anspruch 3, gebacken wird.

7. Brotprodukt, erhalten durch das Verfahren gemäß Anspruch 6.


**Claims**

1. A *Saccharomyces cerevisiae* yeast strain deposited on April 25, 2013, with the CNCM under number I-4743.

2. A baker's yeast obtained by culturing the strain according to claim 1, **characterized in that** it is in a liquid, semi-liquid, compressed or dry form.

3. A process for preparing a baker's dough, comprising a fermentation step with a yeast according to claim 2.

4. A baker's dough obtained according to the process of claim 3.

5. The baker's dough according to claim 4, **characterized in that** it comprises less than 10% of added sugar.

6. A process for preparing a baked breadmaking product, comprising a step of baking a baker's dough obtained according to the process of claim 3.

7. A breadmaking product obtainable by the process according to claim 6.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2012110711 A
- EP 0511108 A
- US 5741695 A

**Littérature non-brevet citée dans la description**

- **G. REED ; T.W. NAGODAWITHANA.** Yeast Technology. Van Nostrand Reinhold, 1991
- **BURROWS ; HARRISON.** *Journal of the Institute of Brewing,* 1959, vol. 65